# EUROPÄISCHE PATENTSCHRIFT

(11) **EP 2 318 057 B1**
(45) Veröffentlichungstag und Bekanntmachung des Hinweises auf die Patenterteilung: **04.03.2015**
(21) Anmeldenummer: 09777806.2
(22) Anmeldetag: 11.08.2009
(51) Int. Cl.: A61L 27/00, A61L 31/14, A61B 17/68, A61B 17/04, A61F 2/44

(54) **IMPLANTAT AUS MAGNESIUM UND VERFAHREN ZU DESSEN HERSTELLUNG**
IMPLANT MADE OF MAGNESIUM AND METHOD FOR THE PRODUCTION THEREOF
IMPLANT EN MAGNÉSIUM ET SON PROCÉDÉ DE FABRICATION

(30) Priorität: 11.08.2008 DE 102008037200
(43) Veröffentlichungstag der Anmeldung: 11.05.2011
(73) Patentinhaber: aap Implantate AG, 12099 Berlin (DE)
(72) Erfinder: WITTE, Frank, 30171 Hannover (DE); HORT, Norbert, 21339 Lüneburg (DE); WOLFSTÄDTER, Marco, 63939 Wörth/Main (DE); FISCHER, hans-Joachim, 12277 Berlin (DE); VOITH, Wolfgang, 78554 Aldingen (DE); FRÖHLICH, Bernd, 34225 Baunatal (DE)
(74) Vertreter: Herden, Andreas F.
(86) Internationale Anmeldenummer: PCT/EP2009/005818
(87) Internationale Veröffentlichungsnummer: WO 2010/017959

(56) Entgegenhaltungen:
- EP-A- 0 875 218
- WO-A-2006/119789
- WO-A-2007/016796
- WO-A-2007/125532
- WO-A-2008/034007
- US-A- 5 205 338
- US-A1- 2003 049 150
- US-A1- 2005 177 238

## Beschreibung

### Gebiet der Erfindung

### Hintergrund der Erfindung

Bioresorbierbare Implantate, insbesondere Implantate aus Magnesium beziehungsweise einer Magnesiumlegierung sind bekannt. Es ist insbesondere bekannt, dass Magnesium sich positiv auf die Heilung von Knochen auswirkt und sich im Körper langsam zersetzt, so dass mittels Magnesium bioresorbierbare Implantate hergestellt werden können.

Insbesondere gibt es Schrauben, Drähte, Nägel etc., welche im Wesentlichen aus Magnesium bestehen.

Nachteilig an bekannten Magnesiumimplantaten ist, dass die mechanischen Eigenschaften eines Implantats, insbesondere von Schrauben, bereits nach der Zersetzung des Implantats bis in eine geringe Tiefe derart geschwächt werden, dass das Implantat seine mechanische Wirkung verliert. Das dann verbleibende Restimplantat, also der Kern des Implantats verbleibt sodann im Körper und unterliegt einem relativ langen Zerfallsprozess, da ein weiterer korrosiver Angriff immer nur an der Oberfläche erfolgt.

Ein weiterer Nachteil von bekannten Magnesiumimplantaten ist die schwierige Herstellung des Implantats mit spanenden Bearbeitungsverfahren. Bei der spanenden Bearbeitung von Magnesiumlegierungen besteht immer eine hohe Brandgefahr. Die Herstellung muss daher entsprechend vorsichtig und aufwändig durchgeführt werden.

Ein poröses Implantat aus Magnesium nach der Präambel des Anspruchs 1 ist aus der europäischen Patentanmeldung EP-A2-0875218 bekannt. Sowohl die Herstellung von Implantaten aus Magnesium durch Druckgiessen, als auch die Herstellung pöroser Implantate aus Magnesium durch Sintern, sind aus der PCT Patentanmeldung WO-A2-2007/125532 bekannt.

### Aufgabe der Erfindung

Der Erfindung liegt demgegenüber die Aufgabe zugrunde, ein Implantat mit verbesserten Eigenschaften für den Behandlungserfolg bereitzustellen, welches sich zudem einfach herstellen lässt.

Eine weitere Aufgabe der Erfindung ist es, ein Implantat bereitstellen zu können, bei welchem die mechanischen Eigenschaften des Implantats während des Abbauprozesses über eine verhältnismäßig lange Zeitdauer erhalten bleiben, bei dem also der Zersetzungsprozess des Restimplantats, welches ohnehin seine Funktion verloren hat, einen möglichst geringen Anteil der gesamten Verweilzeit im Körper einnimmt.

Eine weitere Aufgabe der Erfindung ist es, die Masse an bioresorbierbarem Material, welches im Körper abgebaut werden muss, zu reduzieren.

Eine weitere Aufgabe einer Ausführung ist es, eine Magnesiumlegierung für medizinische Anwendungen bereitzustellen, welche eine hohe Verträglichkeit bei gleichzeitig guten mechanischen Eigenschafen besitzt. Insbesondere ist es eine Aufgabe dieser Ausführung, den Anteil von Zink, welches im Körper bei Überdosierung Entzündungen auslösen kann, aber zur Verbesserung der mechanischen Eigenschaften in Magnesiumlegierungen verwendet wird, zu reduzieren.

### Zusammenfassung der Erfindung

Die Aufgabe der Erfindung wird bereits durch ein Implantat aus Magnesium oder einer Magnesiumlegierung nach Anspruch 1 gelöst.

Bevorzugte Ausführungsformen und Weiterbildungen der Erfindung sind den jeweiligen Unteransprüchen zu entnehmen.

Die Erfindung betrifft zum einen ein Implantat aus Magnesiumdruckguss oder einer Magnesiumdruckgusslegierung, wobei das Implantat zumindest abschnittsweise porös ist und die Porosität zumindest abschnittsweise von außen nach innen zunimmt. Gemäß der Erfindung nimmt die Porosität von der oder den Oberflächen des Implantats in Richtung Kern des Implantats also zu. Dies hat zur Folge, dass der Abbauprozess an der weniger porösen Oberfläche im Verhältnis zum Kern des Implantats verlangsamt ist. Nach dem Einsetzen des Implantats beginnt die Zersetzung also relativ langsam, nachdem sich die oberflächennahen Bereiche aber aufgelöst haben, wird der verbleibende Restkern des Implantats für eine schnelle Zersetzung freigegeben. Die Oberfläche weist dagegen vorzugsweise eine Gusshaut auf, die einen korrosiven Angriff in der Anfangszeit nach dem Einsetzen verlangsamt.

Das Implantat behält somit für einen im Verhältnis längeren Zeitraum des gesamten Abbauprozesses die erforderlichen mechanischen Mindesteigenschaften.

Bei einer bevorzugten Ausführungsform der Erfindung ist die Oberfläche des Implantats im Wesentlichen frei von offenen Poren, insbesondere weist die Oberfläche weniger als 3 offene Poren mit einem Durchmesser von mehr als 100 µm/cm² auf.

Die Erfinder haben herausgefunden, dass bereits eine relativ geringe Porosität zu einem überraschend stark beschleunigten Zersetzungsprozess führt. Bei einer geschlossenen, im Wesentlichen porenfreien, Oberfläche setzt dagegen der Zersetzungsprozess stark verlangsamt ein.

Bei eine weiteren bevorzugten Ausführungsform der Erfindung ist das Implantat derart ausgebildet, dass die Gefügestruktur, also der mittlere Korndurchmesser einer zumindest teilkristallinen Legierung nach innen, also in Richtung des Kerns hin, gröber wird. Eine höhere Korngröße scheint ebenfalls mit einer schnelleren Zersetzung im Körper einherzugehen.

Vorzugsweise beträgt die Porosität in einem ersten oberflächennahen Bereich, welcher beispielsweise mit einer Tiefe von maximal 0,5 mm definiert ist, weniger als 3%, besonders bevorzugt weniger als 2%.

In einem oberflächenfernen Bereich, welcher insbesondere durch eine Tiefe von mehr als 0,6 mm von der Oberfläche aus gesehen definiert werden kann, beträgt vorzugsweise der Porositätsgrad mehr als 3%, besonders bevorzugt mehr als 5%. Unter Porosität wird die geschlossene Porosität verstanden, also der Anteil offener und geschlossener Poren. Der Porositätsgrad des Implantats kann 1 bis 40%, vorzugsweise 2 bis 8% betragen.

Technisch möglich sind Porositäten bis etwa 40%. Es hat sich aber gezeigt, dass bereits relativ geringe Porositätsgrade, insbesondere von mehr als 3% den Abbauprozess des verbleibenden Restimplantats erheblich beschleunigen.

Bei einer Weiterbildung der Erfindung umfasst das Implantat eine Magnesiumlegierung mit einem Anteil an Yttrium von 0,5 bis 10% (%-Angaben, soweit nicht anders angegeben immer in Gewichts-%), vorzugsweise von 1 bis 9% und besonders bevorzugt von 3 bis 5%. Yttrium dient als Zuschlagsstoff vor allem der Verzögerung der Korrosion des Implantats. Über den Anteil an Yttrium kann also die gewünschte Einsatzzeit des Implantats, bis dieses durch Zersetzung seine Eigenschaften verliert, eingestellt werden.

Vorzugsweise umfasst die Magnesiumlegierung einen Anteil an sonstigen seltenen Erden, also Seltenerdenmetallen außer Yttrium, von weniger als 2%, besonders bevorzugt von weniger als 1%.

Der Anteil an den Elementen Gd, Tb, Dy, Ho, Er, Tm, Yb und Lu an der Legierung, welche eine hohe Löslichkeit aufweisen, beträgt bei einer bevorzugten Ausführungsform der Erfindung weniger als jeweils 0,4%, in Summe weniger als 0,7%.

Der Anteil der Elemente Nd, La, Ce, Pr, Sm und Eu, welche eine relativ hohe Löslichkeit besitzen, beträgt bei einer bevorzugten Ausführungsform der Erfindung weniger als jeweils 0,15%, in Summe weniger als 0,4%.

So konnte eine Legierung mit homogenem Ausscheideverhalten beim Gießen bereitgestellt werden.

Zur Vermeidung von teils unerwünschten Nebenwirkungen weist das Implantat vorzugsweise einen Anteil zumindest eines der Elemente Al, Zn, Si, Mn, Ca, Zr, Li, Sn, Sr und P von weniger als jeweils 0,2%, in Summe weniger als 0,4% auf.

Weiter umfasst das Implantat vorzugsweise von den Elementen Ag, Au, Cu, Fe und Ni jeweils weniger als 50 ppm.

Bei einer alternativen Ausführungsform der Erfindung ist, je nach Indikation, aber auch vorgesehen, Silber, insbesondere in atomarer oder nanopartikulärer Form zur Erzielung von antiseptischen Eigenschaften zuzugeben.

Der Anteil an Berillium beträgt vorzugsweise weniger als 4 ppm.

Bei einer Weiterbildung der Erfindung ist das Implantat zumindest abschnittsweise beschichtet.

So ist beispielsweise vorgesehen, eine Beschichtung zu verwenden, welche ebenfalls bioresorbierbar ist, sich also im Körper abbaut.

Insbesondere sind biokompatible Polymere, sich langsam auflösende Oxidschichten oder degradierbare Beschichtungen aus Hydroxylapatit oder anderen Calciumphophatsalzen vorgesehen. Auch Beschichtungen aus Polymeren, zum Beispiel Poly-L-milchsäure, Polycaprolactone oder Fluoridbeschichtungen lösen sich innerhalb einer bestimmten Verweildauer auf.

Über eine Beschichtung kann in der Anfangszeit nach dem Einsetzen des Implantats der Abbau nahezu vollständig verhindert werden. Das Implantat behält somit in der Anfangszeit seine mechanischen Eigenschaften.

Bei einer weiteren Ausführungsform der Erfindung wird eine Beschichtung verwendet, die sich bei einer Temperatur, welche oberhalb der Körpertemperatur liegt, zersetzt oder auflöst. So ist insbesondere vorstellbar, ein eingesetztes Implantat induktiv auf eine Temperatur zwischen 40 und 60°C zu erhitzen, wobei sich die Beschichtung abbaut und das verbleibende Magnesiumimplantat zur Zersetzung freigibt.

Bei einer Weiterbildung der Erfindung weist das Implantat eine gestrahlte Oberfläche auf. So können zum Einen Grate entfernt werden. Zum Anderen kann Strahlen zu einer Verdichtung der Oberfläche führen, was den anfänglichen Zersetzungsprozess verlangsamt.

Bei einer bevorzugten Aufgabe der Erfindung ist das Implantat einstückig ausgebildet.

Das Herstellungsverfahren für die erfindungsgemäßen Implantate ist ein Druckgussverfahren.

Die Erfinder haben herausgefunden, dass es mittels eines Druckgussverfahrens möglich ist, auf sehr wirtschaftliche Weise Implantate mit porösem Kern, aber im Wesentlichen porenfreier Oberfläche bereitzustellen.

Die Schmelze wird dabei mit hohem Druck in eine Form gepresst. Die Form ist dabei vorzugsweise nicht evakuiert, wie es bei einigen Vakuum unterstützen Druckgussverfahren der Fall ist. Die in der Form befindlichen Gase führen zu einer Verwirbelung der einströmenden Schmelze, bei welcher Gas, insbesondere Luft, eingeschlossen wird und zur Porosität des Implantats führt, wobei die Oberfläche des Implantats im Wesentlichen porenfrei und geschlossen bleibt.
Die Schmelze wird vorzugsweise bei Durchführung des Druckgussverfahrens mit hohem Druck in die Form gepresst, insbesondere mit über 20, vorzugsweise über 100 bar.

Vorzugsweise wird die Schmelze sehr dünnflüssig mit hoher Temperatur verarbeitet. Insbesondere beträgt die Gießtemperatur über 600, vorzugsweise über 700 °C.

Die Gießgeschwindigkeit beträgt bei einer bevorzugten Ausführungsform der Erfindung über 5, vorzugsweise über 20 cm/s.

Die Erfinder haben heraus gefunden, dass sich bei hoher Temperatur und hohem Druck auch Legierungen mit geringem Aluminiumanteil und, mit Ausnahme von Yttrium, mit geringem Anteil weiterer Seltenerden verarbeiten lassen.

Anders als bei bekannten Druckgussverfahren für nichtmedizinische Anwendungen kommt es durch das schlechtere Fließverhalten der vorzugsweise verwendeten Legierung zur Bildung poröser Strukturen im Inneren des Implantats. Diese porösen Strukturen, welche bei nichtmedizinischen Anwendungen unbedingt zu vermeiden sind, führen bei dem bioresorbierbaren Implantat zu den beschriebenen verbesserten Eigenschaften.

Neben einer Gratentfernung, beispielsweise durch Strahlen, kann das Implantat alternativ oder zusätzlich auch durch Zündung einer Explosion in einer Trommel, in welcher sich das Implantat befindet, entgrated werden.

Die Erfindung eignet sich zur Ausbildung von Implantaten für verschiedenste Einsatzzwecke, insbesondere Schrauben, Fadenanker, also Anker, mit welchen Wundnahtfäden befestigt werden, sowie für Wundnahtanker, also eine Ankeranordnung zum Befestigen von Weichgewebe an Knochen.

Diese Offenbarung betrifft des Weiteren eine Magnesiumlegierung für ein Implantat, insbesondere eine Magnesiumdruckgusslegierung. Die Magnesiumlegierung weist 1 bis 9% Yttrium, sowie zwischen 0,1 und 1,5 % weitere Seltenerdmetalle auf, wobei der Anteil an Zink weniger als 0,4% beträgt.

Die Erfinder haben herausgefunden, dass über eine geeignete Auswahl an Seltenerdmetallen zusätzlich zum Yttrium die mechanischen Eigenschaften sich derart einstellen lassen, dass das Implantat auch ohne Zusatz von Zink die notwendige Festigkeit aufweist. Auch auf einen hohen Anteil an Al und weiterer Seltenerdemetalle kann verzichtet werden. Die Legierung hat daher eine gute Verträglichkeit.

Insbesondere ist eine Magnesiumlegierung mit folgenden weiteren Bestandteilen vorgesehen:
Gd, Tb, Dy, Ho, Er, Tm, Yb und Lu weniger als jeweils 0,4%, in Summe zwischen 0,1 und 0,7%
Nd, La, Ce, Pr, Sm und Eu weniger als jeweils 0,15%, in Summe zwischen 0,05 und 0,3%;
Al, Zn, Si, Mn, Ca, Zr, Li, Sn, Sr und P weniger als jeweils 0,2%, in Summe zwischen 0,05 und 0,3%;
Ag, Au, Cu, Fe und Ni weniger als jeweils 50 ppm;
Be weniger als 4 ppm.

So lässt sich eine kristalline Druckgusslegierung mit Korngrößen zwischen 2 und 2000 µm bereitstellen. Insbesondere ist es möglich, eine mikrokristalline Druckgusslegierung bereitstellen zu können.

Die Magnesiumlegierung erreicht eine Streckgrenze R_{p0,2} von mehr als 70, vorzugsweise von mehr als 75 Mpa.

Die Bruchdehnung Rₘ beträgt mehr als 90, vorzugsweise mehr als 100 Mpa, die Bruchdehnung A mehr als 3%. Eine Ausführung bezieht sich des weiteren auf ein Implantat aus Magnesium oder eine Magnesiumlegierung, wobei das Implantat zumindest abschnittsweise beschichtet ist.

Das Implantat besteht somit aus einem biodegradierbarem Kern aus Magnesium oder einer Magnesiumlegierung, welcher erst nach Auslösen oder Abschmelzen der Beschichtung zum Abbau freigegeben wird. Die mechanischen Eigenschaften des Implantats bleiben somit nach dem Einsetzen über einen verhältnismäßig langen Zeitraum erhalten. Gemäß der Erfindung ist der Kern zumindest abschnittsweise porös ausgebildet, was den Abbau erheblich beschleunigt, sobald die Beschichtung nicht mehr vorhanden ist.

Als Beschichtungen sind beispielsweise die bereits genannten biokompatible Polymere, zum Beispiel Poly-L-milchsäure, Polycaprolactone, sich langsam auflösende Oxidschichten oder degradierbare Beschichtungen aus Hydroxylapatit oder anderen Calciumphophatsalzen oder Fluoridbeschichtungen vorgesehen.

Bei einer Weiterbildung der Erfindung weist das Implantat zumindest abschnittsweise eine Beschichtung auf, die sich bei Temperaturen oberhalb der Körpertemperatur zersetzt oder ablöst. Dabei handelt es sich insbesondere um ein Polymer, insbesondere ein Polyethylenoxid oder ein Polylactid.

Die Erwärmung kann beispielsweise von außen durch elektromagnetische Wellen, insbesondere Radio- oder Mikrowellenenergie, durch Induktion oder durch Ultraschalleintrag erfolgen.

Um das angrenzende Gewebe nicht zu schädigen, weist die Beschichtung vorzugsweise eine Schmelztemperatur zwischen 20 und 60 °C auf.

### Beschreibung der Zeichnungen

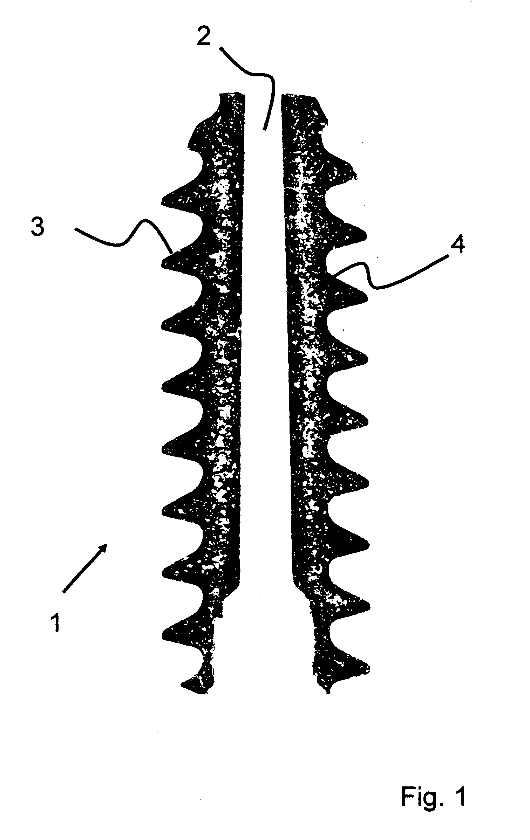

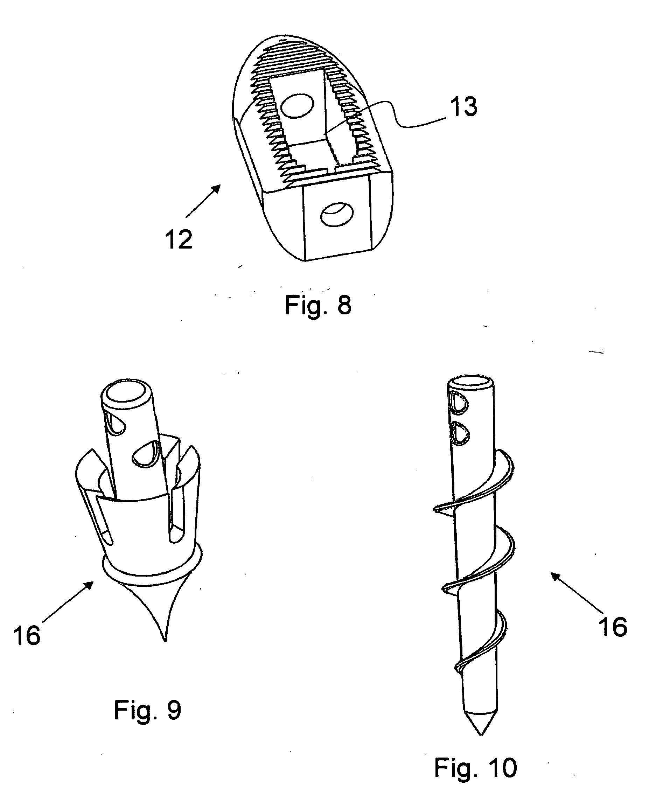

Die Erfindung soll im Folgenden anhand der Zeichnungen Fig. 1 bis Fig. 10 näher erläutert werden.

Fig. 1 zeigt schematisch den Schnitt durch ein Implantat 1, hier ausgebildet als Interferenzschraube. Die Interferenzschraube hat in diesem Beispiel mittig eine Durchführung 2.

Das Implantat 1 ist mittels eines Druckgussverfahrens aus einer Magnesiumlegierung hergestellt und weist eine im Wesentlichen porenfreie und geschlossene Oberfläche 3 auf.

Innen, also in einem oberflächenfernen Bereich 4, besitzt das Implantat 1 eine wesentlich höhere Porosität als an der Oberfläche 3.

Da die Durchführung 2 nicht mittels einer Bohrung sondern bereits durch die Druckgussform vorgegeben wurde, besitzt auch die Oberfläche der Durchführung 2 eine im Wesentlichen porenfreie und geschlossene Oberfläche.

Nach dem Einsetzen des Implantats in den Körper ist die Zersetzungsgeschwindigkeit an der Oberfläche 3 des Implantats erheblich reduziert, gegenüber der Zersetzungsgeschwindigkeit im Kern 4 des Implantats.

So kann ein Implantat bereitgestellt werden, welches über einen verhältnismäßig langen Zeitraum die erforderlichen mechanischen Eigenschaften beibehält.

Das Implantat 1 kann eine Beschichtung (nicht dargestellt) aufweisen, welche einen Abbau des Implantats 1 nach dem Einsetzen im Wesentlichen verhindert. Die Beschichtung kann zu einem späteren Zeitpunkt durch Einkopplung von elektromagnetischen Wellen oder durch Induktion abgeschmolzen werden. So wird das eingesetzte Implantat zum Abbau frei gegeben.

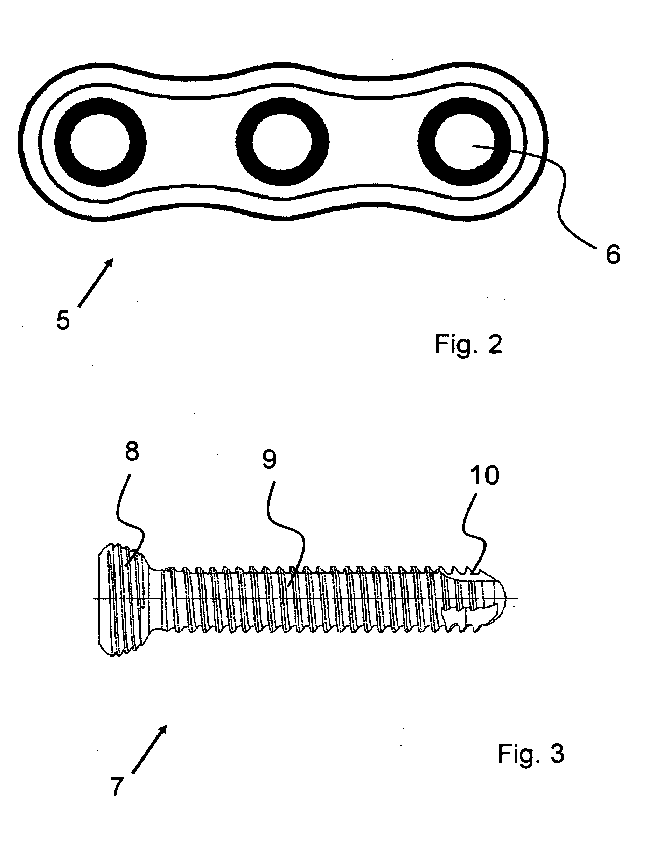

Fig. 2 zeigt schematisch eine Platte 5, wie sie beispielsweise zum Zusammenfügen von Knochenfragmenten verwendet wird.

Zur Befestigung weist die Platte 5 zumindest ein Gewinde 6 oder alternativ eine Bohrung auf, durch das oder die eine Schraube in den Knochen gedreht wird.

Das Gewinde 6 wird vorzugsweise bereits während des Druckgusses mittels eines in die Druckgussform eingeführten Gewindestiftes geformt, welcher beim Auswerfen der Platte 5 heraus gespindelt wird. So ist auch die Oberfläche des Gewindes 6 mit einer Gusshaut versehen. Alternativ kann das Gewinde 6 auch geschnitten werden. Dabei wird zwar die Gusshaut zerstört. Nach dem Einsetzen ist das Gewinde 6 aber ohnehin von einer Schraube geschützt.

Fig. 3 zeigt schematisch eine Schraube 7, welche beispielsweise zum Fixieren der in Fig. 2 dargestellten Platte dient. Es handelt sich um ein winkelstabiles System, bei welchem der Kopf 8 der Schraube 7 ein Außengewinde zum Eingriff in das Gewinde der Platte aufweist. Der Schraubenkörper 9 ist mit einem Gewinde zum Eindrehen in den Knochen versehen, welches an der Spitze einen selbstschneidenden Abschnitt 10 aufweist.

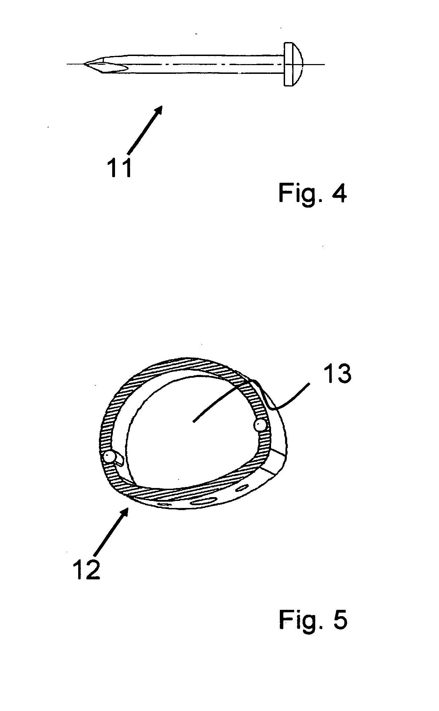

Fig. 4 zeigt schematisch einen Pin 11, welcher mittels eines entsprechenden Werkzeugs eingesetzt wird. Auch dieser Pin 11 wurde mittels eines Druckgussverfahrens hergestellt und ist im Inneren porös. Der Pin 11 ist insbesondere für kleinere Frakturen vorgesehen.

Fig. 5 zeigt einen Cage 12 zum Versteifen von Wirbelkörpern. Der Cage 12 besteht auf einem im Wesentlichen rohrartigen Abschnitt, der in seinem Inneren 13 mit Knochengranulat gefüllt ist. Das Knochengranulat führt zur Bildung von Knochen, so dass die Wirbelkörper nach der Degradierung des Abschnitts aus einer Magnesiumlegierung miteinander verwachsen sind.

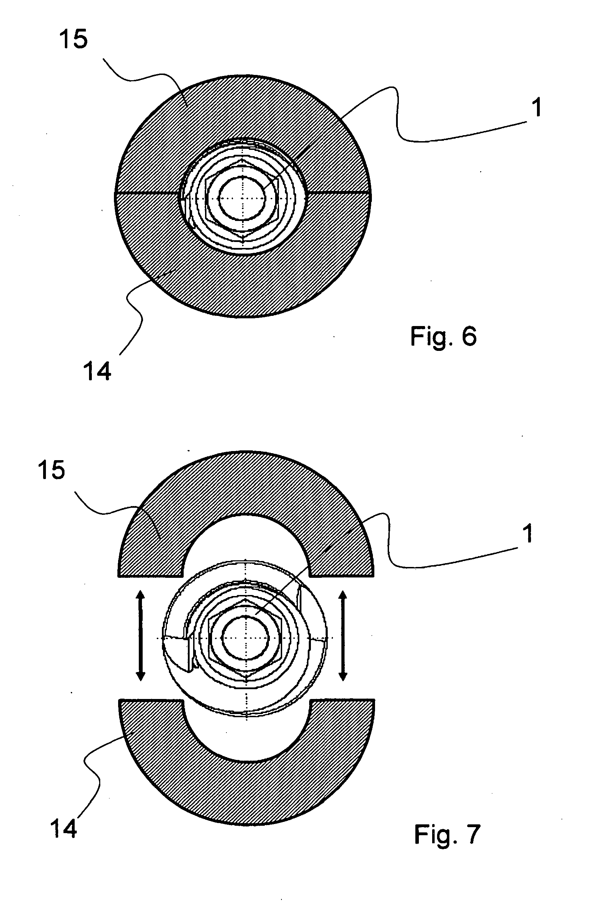

Bezug nehmend auf Fig. 6 und 7 soll schematisch ein Verfahren zur Herstellung eines Implantats 1 erläutert werden.

Fig. 7 zeigt eine Druckgussform in einer Schnittansicht, die ein Oberteil 15 und ein Unterteil 14 aufweist. In diese Form wird eine Yttrium-haltige Magnesiumlegierung mit hoher Temperatur und hohem Druck gepresst. Die Schmelze erstarrt innerhalb von Sekundenbruchteilen. Dabei bildet sich an der Oberfläche des Implantats 1 eine feinkörnige Gusshaut aus, wohingegen sich im Inneren des Implantats vermutlich aufgrund von Verwirbelungen und des verhältnismäßig schlechten Fließverhaltens der verwendeten Legierung eine poröse Struktur mit gröberem Gefüge ausbildet.

Sodann wird, wie in Fig. 7 dargestellt, die Form, bestehend aus Oberteil 15 und Unterteil 14, geöffnet und das Implantat 1 ausgeworfen.

Es versteht sich, dass die Form nur sehr schematisch dargestellt ist und diese in der Praxis noch weitere Teile und Komponenten umfasst. Insbesondere ist auch eine zumindest vierteilige Form vorgesehen.

Fig. 8 zeigt eine alternative Ausführungsform eines Cage 12, welcher zum Einsatz in der Halswirbelsäule ausgebildet ist. Auch dieser Cage 12 weist eine Aussparung 13 auf, welche mit Knochengranulat gefüllt sein kann.

Fig. 9 und Fig. 10 zeigen verschiedene Ausführungsformen eines Fadenankers 16.

## Patentansprüche

1. Implantat aus Magnesium oder einer Magnesiumlegierung, welches zumindest abschnittsweise porös ist, wobei die Porosität zumindest abschnittsweise von außen nach innen zunimmt **dadurch gekennzeichnet, dass** es sich um ein Implantat aus Magnesiumdruckguss oder einer Magnesiumdruckgusslegierung handelt.

2. Implantat nach dem vorstehenden Anspruch, **dadurch gekennzeichnet, dass** zumindest eine Oberfläche des Implantats im Wesentlichen frei von offenen Poren ist.

3. Implantat nach dem vorstehenden Anspruch, **dadurch gekennzeichnet, dass** die Oberfläche weniger als 3 offene Poren mit einem Durchmesser von mehr als 100 µm pro cm² aufweist.

4. Implantat nach einem der vorstehenden Ansprüche, **dadurch gekennzeichnet, dass** die Gefügestruktur des Magnesiums oder der Magnesiumlegierung nach innen hin gröber wird.

5. Implantat nach einem der vorstehenden Ansprüche, **dadurch gekennzeichnet, dass** der Porositätsgrad in einem ersten, oberflächennahen Bereich weniger als 3%, vorzugsweise weniger als 2% beträgt.

6. Implantat nach dem vorstehenden Anspruch, **dadurch gekennzeichnet, dass** der oberflächennahe Bereich durch eine Tiefe von maximal 0,5 mm definiert ist.

7. Implantat nach einem der vorstehenden Ansprüche, **dadurch gekennzeichnet, dass** der Porositätsgrad in einem weiteren, oberflächenfernen Bereich mehr als 3%, vorzugsweise mehr als 5% beträgt und wobei der oberflächenferne Bereich vorzugsweise durch eine Tiefe von mehr 0,6 mm definiert ist.

8. Implantat nach einem der vorstehenden Ansprüche, **dadurch gekennzeichnet, dass** der Porositätsgrad des Implatats 1 bis 40%, vorzugsweise 2 bis 8% beträgt.

9. Implantat nach einem der vorstehenden Ansprüche, **dadurch gekennzeichnet, dass** das Implantat zumindest abschnittsweise beschichtet ist.

10. Verfahren zum Herstellen eines Implantats, **dadurch gekennzeichnet, dass** eine Magnesiumlegierung mittels eines Druckgussverfahrens zu einem zumindest abschnittsweise porösen Implantat geformt wird, wobei die Porosität zumindest abschnittsweise von außen nach innen zunimmt.

11. Verfahren zum Herstellen eines Implantats nach dem vorstehenden Anspruch, **dadurch gekennzeichnet, dass** eine Magnesiumlegierung mit 1 bis 9% Y und zwischen 0,1 und 1,5 % weiteren Seltenerdemetallen verwendet wird.

12. Verfahren zum Herstellen eines Implantats nach einem der vorstehenden Ansprüche, **dadurch gekennzeichnet, dass** der Druck, mit dem die Schmelze bei Durchführung des Druckgussverfahrens in die Form gepresst wird, über 20, vorzugsweise über 100 bar beträgt und/oder die Gießtemperatur über 600, vorzugsweise über 700 °C beträgt und/oder dass die Gießgeschwindigkeit über 5, vorzugsweise über 20 cm/s beträgt.

## Claims

1. An implant made of magnesium or a magnesium alloy, which is porous at least in portions thereof, with the porosity increasing from the outside inwards, at least in portions thereof, **characterized in that** the implant is a die-cast magnesium implant or a die-cast magnesium alloy implant.

2. The implant according to the preceding claim, **characterized in that** at least one surface of the implant is substantially free of open pores.

3. The implant according to the preceding claim, **characterized in that** the surface has less than 3 open pores of a diameter of more than 100 µm per cm².

4. The implant according to any of the preceding claims, **characterized in that** the coarseness of the microstructure of the magnesium or the magnesium alloy increases inwardly.

5. The implant according to any of the preceding claims, **characterized in that** in a first, near-surface region the degree of porosity is less than 3 %, preferably less than 2 %.

6. The implant according to the preceding claim, **characterized in that** the near-surface region is defined by a depth of at most 0.5 mm.

7. The implant according to any of the preceding claims, **characterized in that** in a further region spaced from the surface the degree of porosity is more than 3 %, preferably more than 5 %, and wherein the region spaced from the surface is preferably defined by a depth of more than 0.6 mm.

8. The implant according to any of the preceding claims, **characterized in that** the degree of porosity of the implant is from 1 to 40 %, preferably from 2 to 8 %.

9. The implant according to any of the preceding claims, **characterized in that** the implant is coated, at least in portions thereof.

10. A method for producing an implant, **characterized in that** a magnesium alloy is formed into an implant that is porous at least in portions thereof using a die-casting process, wherein the porosity increases from the outside inwards, at least in portions.

11. The method for producing an implant according to the preceding claim, **characterized in that** a magnesium alloy is used that includes from 1 to 9 % of Y and from 0.1 to 1.5 % of other rare earth metals.

12. The method for producing an implant according to any of the preceding claims, **characterized in that** the pressure used for pressing the melt into the mold when performing the die-casting process, is more than 20, preferably more than 100 bar, and/or that the casting temperature is above 600, preferably above 700 °C, and/or that the casting speed is more than 5, preferably more than 20 cm/s.

## Revendications

1. Implant en magnésium ou en alliage de magnésium, lequel est au moins partiellement poreux, la porosité croissant au moins partiellement de l'extérieur vers l'intérieur, **caractérisé en ce qu'**il s'agit d'un implant en magnésium à coulée sous pression ou en alliage de magnésium à coulée sous pression.

2. Implant selon la revendication précédente, **caractérisé en ce qu'**au moins une surface de l'implant est sensiblement exempte de pores ouverts.

3. Implant selon la revendication précédente, **caractérisé en ce que** la surface présente moins de 3 pores ouverts ayant un diamètre supérieur à 100 µm par cm².

4. Implant selon l'une des revendications précédentes, **caractérisé en ce que** la structure du magnésium ou de l'alliage de magnésium est de plus en plus grossière en allant vers l'intérieur.

5. Implant selon l'une des revendications précédentes, **caractérisé en ce que** le degré de porosité est inférieur à 3 %, préférentiellement inférieur à 2 % dans une première zone proche de la surface.

6. Implant selon la revendication précédente, **caractérisé en ce que** la zone proche de la surface est définie par une profondeur maximale de 0,5 mm.

7. Implant selon l'une des revendications précédentes, **caractérisé en ce que** le degré de porosité est supérieur à 3 %, préférentiellement supérieur à 5 % dans une autre zone distante de la surface, ladite zone distante de la surface étant définie par une profondeur supérieure à 0,6 mm.

8. Implant selon l'une des revendications précédentes, **caractérisé en ce que** le degré de porosité de l'implant est compris entre 1 et 40 %, préférentiellement entre 2 et 8 %.

9. Implant selon l'une des revendications précédentes, ledit implant étant **caractérisé en ce qu'**il est au moins partiellement revêtu.

10. Procédé de fabrication d'un implant, **caractérisé en ce qu'**un alliage de magnésium est moulé au moyen d'un procédé de coulée sous pression pour former un implant au moins partiellement poreux, la porosité croissant au moins partiellement de l'extérieur vers l'intérieur.

11. Procédé de fabrication d'un implant selon la revendication précédente, **caractérisé en ce qu'**un alliage de magnésium comprenant de 1 à 9 % d'Y et de 0,1 à 1,5 % d'autres métaux de terres rares est utilisé.

12. Procédé de fabrication d'un implant selon l'une des revendications précédentes, **caractérisé en ce que** la pression avec laquelle la fonte est pressée dans le moule lors de l'exécution du procédé de coulée sous pression est supérieure à 20, préférentiellement à 100 bar, et/ou **en ce que** la température de coulée est supérieure à 600, préférentiellement à 700 °C, et/ou **en ce que** la vitesse de coulée est supérieure à 5, préférentiellement à 20 cm/s.
